# FASCICULE DE BREVET EUROPEEN

(11) **EP 1 248 650 B1**
(45) Date de publication et mention de la délivrance du brevet: **29.11.2006**
(21) Numéro de dépôt: 01907651.2
(22) Date de dépôt: 19.01.2001
(51) Int. Cl.: A61K 39/39, A61K 47/18, A61P 31/12, C12N 15/38

(54) **VACCINS ADN AMELIORES POUR HERPESVIRUS-1 BOVIN**
VERBESSERTE DNS IMPFSTOFFE FÜR RINDER-HERPES VIRUS 1
IMPROVED DNA VACCINES FOR BOVINE HERPESVIRUS-1

(30) Priorité: 21.01.2000 FR 0000798
(43) Date de publication de la demande: 16.10.2002
(73) Titulaire: MERIAL, 69002 Lyon (FR)
(72) Inventeur: AUDONNET, Jean-Christophe, Francis, F-69006 Lyon (FR); FISCHER, Laurent, Bernard, F-69110 Sainte-Foy Les Lyon (FR); BARZU-LE-ROUX, Simona, F-69210 Lentilly (FR)
(74) Mandataire: Nargolwalla, Cyra
(86) Numéro de dépôt international: PCT/FR2001/000187
(87) Numéro de publication internationale: WO 2001/052888

(56) Documents cités:
- WO-A-94/01133
- WO-A-94/27435
- WO-A-98/03196
- WO-A-98/03658
- WO-A-98/40499
- WO-A1-96/34109
- FR-A- 2 751 229
- US-A- 5 719 131
- GAO YI ET AL: "Truncated bovine herpesvirus-1 glycoprotein I (gpI) initiates a protective local immune response in its natural host" VACCINE,GB,BUTTERWORTH SCIENTIFIC. GUILDFORD, vol. 12, no. 2, 1994, pages 145-152, XP002133950 ISSN: 0264-410X
- WHEELER C J ET AL: "CONVERTING AN ALCOHOL TO AN AMINE IN A CATIONIC LIPID DRAMATICALLY ALTERS TO CO-LIPID REQUIREMENT, CELLULAR TRANSFECTION ACTIVITY AND THE ULTRASTRUCTURE OF DNA-CYTOFECTIN COMPLEXES" BIOCHIMICA ET BIOPHYSICA ACTA,NL,AMSTERDAM, vol. 1280, no. 1, 1996, pages 1-11, XP002035803 ISSN: 0006-3002
- FELGNER J H ET AL: "ENHANCED GENE DELIVERY AND MECHANISM STUDIES WITH A NOVEL SERIES OF CATIONIC LIPID FORMULATIONS" JOURNAL OF BIOLOGICAL CHEMISTRY,US,AMERICAN SOCIETY OF BIOLOGICAL CHEMISTS, BALTIMORE, MD, vol. 269, no. 4, 28 janvier 1994 (1994-01-28), pages 2550-2561, XP002010485 ISSN: 0021-9258
- HADDAD DIANA ET AL: "Comparative study of DNA-based immunization vectors: Effect of secretion signals on the antibody responses in mice" BIOSIS, XP002133953
- LI XIAOMAO ET AL: "Protection against respiratory syncytial virus infection by DNA immunization" JOURNAL OF EXPERIMENTAL MEDICINE,TOKYO,JP, vol. 188, no. 4, 17 août 1998 (1998-08-17), pages 681-688, XP002133951 ISSN: 0022-1007
- ZABNER ET AL.: "CELLULAR AND MOLECULAR BARRIERS TO GENE TRANSFER BY A CATIONIC LIPID" JOURNAL OF BIOLOGICAL CHEMISTRY, vol. 270, no. 32, 11 août 1995 (1995-08-11), pages 18997-19007,

## Description

La présente invention a trait à des vaccins ADN améliorés pour les bovins.

L'utilisation de molécules d'acide désoxyribonucléique (ADN) pour la vaccination est connue depuis le début des années 90 (Wolf *et al.* Science 1990. **247.** 1465-1468). Cette technique de vaccination induit une immunité cellulaire et humorale après la transfection *in vivo* de cellules du sujet à vacciner par des molécules d'ADN ou d'ARN codant pour des protéines immunologiquement actives.

Un vaccin ADN se compose d'au moins un plasmide pouvant être exprimé par la machinerie cellulaire du sujet à vacciner et d'un véhicule ou d'un excipient pharmaceutiquement acceptable. La séquence nucléotidique de ce plasmide code pour, entre autres, un ou plusieurs immunogènes, tels que des protéines ou glycoprotéines capables d'induire, chez le sujet à vacciner, une réponse immunitaire cellulaire (mobilisation des lymphocytes T) et humorale (stimulation de la production d'anticorps spécifiquement dirigés contre l'immunogène) (Davis H. L. Current Opinion Biotech. 1997. 8. 635-640).

Tous les immunogènes provenant d'un pathogène ne sont pas des antigènes assez efficaces naturellement pour induire une réponse immunitaire protectrice optimale chez l'animal à vacciner. Il est donc nécessaire d'améliorer la réponse immunitaire.

Différentes voies d'administration du vaccin ADN sont proposées (intrapéritonéale, intraveineuse, intramusculaire, sous-cutanée, intradermique, muqueuse, etc.). Différents moyens d'administration ont également été proposés, notamment des particules d'or enrobées d'ADN et projetées de façon à pénétrer dans les cellules de la peau du sujet à vacciner (Tang *et al.* Nature 1992. **356.** 152-154) et les injecteurs par jet liquide permettant de transfecter à la fois des cellules de la peau et des cellules des tissus sous-jacents (Furth *et al.* Analytical Bioch. 1992. 205. 365-368).

Des composés chimiques ont été utilisés pour la transfection *in vitro* d'ADN :
A/ - les lipides cationiques.
   Les lipides cationiques sont eux-mêmes divisés en quatre sous-groupes.
   1) les lipides cationiques contenant des sels d'ammonium quaternaire, comme par exemple le DOTMA (dioléoyloxypropyl-triméthylammonium, produit par Gibco sous le nom de Lipofectine), le DOTAP (triméthyl-2,3-(octadéc-9-ène-oyloxy)-1-propanammonium; Gregoriadis *et al.* FEBS Letters 1997. **402.** 107-110), le DMRIE (N-(2-hydroxyéthyl)-N,N-diméthyl-2,3-bis(tetradécyloxy)-1-propanaminium; WO-A-9634109), le DLRIE (N-(2-hydroxyéthyl)-N,N-diméthyl-2,3-bis(dodécyloxy)-1-propanaminium ; Felgner *et al.* Ann. N Y Acad. Sci. 1995. **772**. 126-139).
      Ces lipides cationiques contenant des sels d'ammonium quaternaire peuvent être associés ou non avec un lipide neutre supplémentaire, tel que le DOPC (dioléoyl-phosphatidyl-choline) ou le DOPE (dioléoyl-phosphatidyl-éthanolamine) (J.P. Behr, Bioconjugate Chemistry 1994. **5.** 382-389).
   2) les lipoamines, comme par exemple le DOGS (dioctadécylamidoglycylspermine, produit par Promega sous le nom de Transfectam; Abdallah *et al.* Biol. Cell. 1995. **85.** 1-7), le DC-Chol (diméthylaminoéthane-carbamoyl-cholestérol ; Gao et Huang, Biochem. Biophys. Res. Commun. 1991. **179.** 280-285), le BGSC (bis-guanidine-spermidine-cholestérol), le BGTC (bis-guanidine-tren-cholestérol) (Vigneron *et al.* Proc. Natl. Acad. Sci. USA 1996. **93**. 9682-9686).
   3) les lipides cationiques contenant des sels d'ammonium quaternaire et des lipoamines, comme par exemple le DOSPA (N,N-diméthyl-N-(2-(sperminecarboxamido)éthyl)-2,3-bis(dioléoyloxy)-1-propanimidium pentahydrochloride, commercialisé par Gibco sous le nom de LipofectAmine® ; Hawley-Nelson *et al.* Focus 1993. **15.** 73-79), le GAP-DLRIE (N-(3-aminopropyl)-N,N-diméthyl-2,3-bis(dodécyloxy)-1-propanaminium; Wheeler *et al.* Proc. Natl. Acad. Sci. USA 1996. **93.** 11454-11459 ; Norman *et al.* Vacine 1997. **15.** 801-803).
   4) les lipides contenant des sels d'amidine, comme par exemple l'ADPDE, l'ADODE (Ruysschaert *et al.* Biochem. Biophys. Res. Commun. 1994. **203.** 1622-1628).
B/ - les polymères, comme par exemple le SuperFect^{™} (molécules de dendrimères activés, produits par Qiagen ; Xu *et al.* Mol. Genet. Metab. 1998. **64.** 193-197), et
C/ - les agents biochimiques, comme par exemple les toxines, notamment les toxines cholériques.

Certains de ces composés ont aussi été utilisés dans la formulation de vaccins ADN avec des résultats plus que mitigés. Les connaissances en matière de transfection *in vitro* ne sont pas transposables à la vaccination ADN où l'objectif final est d'assurer une réaction immunitaire protectrice. Des effets négatifs sur l'induction d'une protection immunitaire efficace ont même été constatés avec des composés connus pour favoriser la transfection *in vitro.* Certains composés chimiques de formulation sont toxiques à hautes doses pour les cellules transfectées.

Dans les travaux de Etchart (Etchart *et al.* J. Gen. Virol. 1997. **78.** 1577-1580), l'utilisation du DOTAP n'a pas eu d'effet adjuvant lors de l'administration du vaccin ADN par la voie intranasale, alors qu'il en a eu par la voie orale. Le DOTAP a également été utilisé dans des vaccins ADN codant pour l'hémagglutinine (HA) du virus de la grippe sur le modèle souris administrés par la voie intranasale (Ban *et al.* Vaccine 1997. **15**. 811-813), mais l'addition de DOTAP a inhibé la réponse immunitaire. L'utilisation de DC-Chol ou de DOTAP/DOPE dans des vaccins ADN codant pour la protéine de surface (S) du virus de l'hépatite B sur le modèle souris administrés par la voie intramusculaire a permis d'augmenter la réponse en anticorps, alors que l'utilisation de la Lipofectine (ou DOTMA) n'a pas augmenté cette réponse (Gregoriadis *et al.* FEBS Letters 1997. **402**. 107-110). Le DC-Chol/DOPE a également été utilisé dans des vaccins ADN contre le virus de l'immunodéficience humaine (HIV, protéine Env) sur le modèle souris, dont l'administration par la voie intramusculaire a induit une réponse immunitaire plus efficace, alors que l'administration par la voie sous-cutanée ou intradermique ne l'a pas augmentée (Ishii *et al.* AIDS Res. Hum. Retro. 1997. **13**. 1421-1428).

L'addition de certaines cytokines, notamment d'interleukines ou d'interférons, peut permettre d'améliorer la réponse immunitaire induite en particulier par les vaccins ADN. Chaque cytokine déclenche une réaction qui lui est propre et oriente plus ou moins la réponse immunitaire vers une réponse cellulaire ou vers une réponse humorale (Pasquini *et al.* Immunol. Cell. Biol. 1997. **75**. 397-401 ; Kim *et al.* J. Interferon Cytokine Res. 1999. **19**. 77-84). Les effets adjuvants d'une cytokine provenant d'une espèce donnée ne sont pas nécessairement les mêmes si le contexte immunitaire varie, notamment si cette cytokine est administrée à une autre espèce, donc dans un système immunitaire hétérologue. L'addition de cytokine peut également n'avoir aucun effet adjuvant, voire aboutir à une inversion de l'effet recherché, c'est-à-dire une diminution ou une inhibition de la réponse immunitaire. Ainsi, un vaccin ADN codant pour une chaîne simple d'une immunoglobuline fusionnée avec le GM-CSF n'augmente pas la réponse immunitaire, alors que l'administration directe chez la souris de cette protéine de fusion est efficace, tout comme l'est l'administration d'une protéine de fusion formée de Fv et de la cytokine IL-1bêta ou l'administration d'un vaccin ADN codant pour cette dernière protéine de fusion (Hakim *et al.* J. Immunol 1996. **157.** 5503-5511). L'utilisation de plasmides co-exprimant la cytokine IL-2 et la protéine d'enveloppe du virus de l'hépatite B dans une conformation fusionnée ou non fusionnée aboutit à l'augmentation des réponses immunitaires humorales et cellulaires (Chow *et al.* J. Virol. 1997. **71.** 169-78). Mais l'utilisation d'un plasmide bicistronique codant pour la glycoprotéine gp120 du virus de l'immunodéficience acquise humaine (HIV-1) et la cytokine IL-2 a induit une réponse immunitaire spécifique anti-gp120 plus faible que celle obtenue par l'utilisation d'un plasmide monocistronique codant uniquement pour gp120 (Barouch *et al.* J. Immunol 1998. **161.** 1875-1882). La co-injection chez la souris de deux vecteurs d'expression, l'un codant pour la glycoprotéine G du virus de la rage, l'autre pour le GM-CSF murin stimule l'activité des lymphocytes B et T, alors que la co-injection avec un plasmide codant pour l'interféron gamma (à la place du GM-CSF murin) a pour résultat une diminution de la réponse immunitaire (Xiang *et al.* Immunity 1995. **2**.129-135).

Certaines modifications au niveau des antigènes, telles que les délétions d'une partie de la séquence nucléotidique codant pour l'antigène, les insertions d'un fragment d'ADN dans la séquence nucléotidique codant pour l'antigène ou dans les régions non traduites en amont ou en aval, peuvent également améliorer l'efficacité des vaccins ADN, notamment en améliorant le niveau d'expression de l'antigène ou sa présentation.

Mais en pratique, les manipulations au niveau de la séquence nucléotidique codant pour l'antigène peuvent entraîner une diminution ou la perte de l'activité immunologique initiale. Ainsi, la délétion du domaine transmembranaire dans le gène codant pour l'antigène G du virus de la rage a diminué le niveau de protection induit chez le modèle souris après administration par la voie intramusculaire d'un vaccin ADN codant pour cet antigène modifié (Xiang *et al.* Virol. 1995. **209**. 569). La délétion du domaine transmembranaire dans le gène codant pour la glycoprotéine gD du virus herpès bovin (BHV) n'a pas permis d'augmenter la réponse en anticorps et n'a induit qu'une protection partielle chez les bovins vaccinés par la voie intramusculaire (van Drunen Little-van den Hurk *et al.* J. Gen. Virol. 1998. **79.** 831-839). Les réponses immunitaires humorales et cellulaires et la protection conférée sont identiques chez des cobayes éprouvés après avoir été immunisés à l'aide soit d'un vaccin ADN codant pour la glycoprotéine GP du virus Ebola, soit d'un vaccin ADN codant pour cette glycoprotéine GP mais sous une forme sécrétée (Xu *et al.* Nature Medicine 1998. **4**. 37-42).

L'insertion de la séquence signal de l'activateur du plasminogène tissulaire humain (en anglais human tissue Plasminogen Activator ou human tPA) dans le gène codant pour l'antigène Pf332 de la malaria n'a pas permis d'augmenter la réponse en anticorps chez la souris vaccinée par voie intramusculaire (Haddad *et al.* FEMS 1997. **18.** 193-202). L'addition en phase d'une séquence tPA au gène codant pour l'antigène VP7 du rotavirus murin n'a pas également permis d'augmenter la réponse en anticorps chez la souris vaccinée par voie intradermique, alors que la protéine de fusion formée de l'antigène VP4 et du tPA a permis cette augmentation, mais sans induire une protection efficace (Chol *et al.* Virology 1998. 250. 230-240).

Les modifications effectuées sur la séquence nucléotidique d'un antigène ne peuvent pas en général être directement transposées à un autre antigène, car les antigènes n'ont pas toujours les mêmes agencements structuraux.

La déposante a pour objectif l'amélioration de l'efficacité de la vaccination ADN. Elle a en particulier pour objectif d'obtenir une meilleure réponse immunitaire et notamment une protection efficace chez les bovins, par la vaccination ADN.

La déposante a pour objectif l'élaboration de vaccins ADN améliorés induisant une réponse immunitaire efficace et protectrice contre le virus herpès bovin de type 1 (BHV-1) encore appelé virus de la rhinotrachéite infectieuse bovine (IBR).

La déposante a également pour objectif l'élaboration de vaccins ADN améliorés permettant d'obtenir une protection immunitaire efficace et protectrice chez le bovin, comprenant BHV-1A.

L'invention a pour objet des vaccins ADN améliorés permettant d'obtenir une protection efficace contre BHV-1 infectant les bovins. Le vaccin ADN est amélioré : soit par sa formulation, soit par l'addition de GM-CSF, soit par l'optimisation du ou des antigènes, soit par des combinaisons de ces solutions.

De préférence, le vaccin ADN est amélioré par sa formulation, et facultativement soit par l'addition de GM-CSF, soit par l'optimisation du ou des antigènes, enfin soit par l'addition de GM-CSF et par l'optimisation du ou des antigènes.

Par définition, le vaccin ADN comprend comme principe actif un plasmide codant pour et exprimant un gène ou fragment de gène, e.g. épitope. Le terme plasmide recouvre une unité de transcription ADN comprenant une séquence polynucléotidique comprenant la séquence du gène à exprimer et les éléments nécessaires à son expression *in vivo.* On préfère la forme plasmide circulaire, super enroulée ou non. La forme linéaire entre également dans le cadre de cette invention.

Chaque plasmide comprend un promoteur apte à assurer, dans les cellules hôtes, l'expression du gène inséré sous sa dépendance. II s'agit en général d'un promoteur eucaryote fort et en particulier d'un promoteur précoce du cytomégalovirus CMV-IE, d'origine humaine ou murine, ou encore éventuellement d'une autre origine telle que rat, cobaye. De manière plus générale, le promoteur est soit d'origine virale, soit d'origine cellulaire. Comme promoteur viral autre que CMV-IE, on peut citer le promoteur précoce ou tardif du virus SV40 ou le promoteur LTR du virus du Sarcome de Rous. Il peut aussi s'agir d'un promoteur de virus dont provient le gène, par exemple le promoteur propre du gène. Comme promoteur cellulaire, on peut citer le promoteur d'un gène du cytosquelette, tel que par exemple le promoteur de la desmine, ou encore le promoteur de l'actine. Lorsque plusieurs gènes sont présents dans le même plasmide, ceux-ci peuvent être présentés dans la même unité de transcription ou dans plusieurs unités différentes.

Suivant une première modalité, les vaccins ADN selon l'invention sont formulés par addition à titre d'adjuvant:

DMRIE (N-(2-hydroxyéthyl)-N,N-diméthyl-2,3-bis(tetradécyloxy)-1-propanammonium ; WO-A-9634109), associé avec un lipide neutre, le DOPE (dioléoyl-phosphatidyl-éthanolamine), pour former le DMRIE-DOPE.

La présente invention a donc pour objet un vaccin ADN contre au moins un pathogène touchant les bovins comprenant au moins un plasmide contenant au moins une séquence nucléotidique codant pour un immunogène BHV-1, dans des conditions permettent l'expression *in vivo* de cette séquence, et un lipide cationique contenant un sel d'ammonium quaternaire, le DMRIE, associé au DOPE.

De préférence, le mélange vecteur recombinant avec cet adjuvant se fait de manière extemporanée et l'on préfère, avant son administration à l'animal, laisser le temps au mélange ainsi constitué de se complexer, par exemple pendant une durée allant de 10 à 60 minutes, notamment de l'ordre de 30 minutes.

Lorsque du DOPE est présent, le ratio molaire DMRIE : DOPE va de préférence de 95 : 5 à 5 : 95, plus particulièrement de 1 : 1.

Le ratio pondéral plasmide : adjuvant DMRIE ou DMRIE-DOPE peut aller notamment de 50 : 1 à 1 : 10, notamment de 10 : 1 à 1 : 5, de préférence de 1 : 1 à 1 : 2.

Suivant une deuxième modalité, on ajoute aux vaccins selon l'invention le GM-CSF (en anglais Granulocyte macrophage - colony stimulating factor ; Clark S. C. *et al.* Science 1987. **230.** 1229 ; Grant S. M. *et al.* Drugs 1992. **53.** 516), ce qui peut se faire par l'incorporation de protéine GM-CSF directement dans la composition vaccinale ou de préférence par l'insertion de la séquence nucléotidique codant pour le GM-CSF dans un vecteur d'expression dans des conditions permettant son expression *in vivo.* Comme vecteur d'expression, on préfère utiliser un plasmide, e.g. le plasmide contenant la séquence nucléotidique codant pour le (ou les) antigène(s) d'intérêt ou un autre plasmide. Pour les bovins le GM-CSF bovin est utilisé.

Suivant une troisième modalité, la (ou les) séquence nucléotidique codant pour l'immunogène sont sous une forme optimisée. Par optimisation, on entend toute modification de la séquence nucléotidique, notamment se manifestant au moins par un niveau d'expression plus élevé de cette séquence nucléotidique, et/ou par une augmentation de la stabilité de l'ARN messager codant pour cet antigène, et/ou par la sécrétion provoquée de cet antigène dans le milieu extracellulaire, et ayant pour conséquence directe ou indirecte une augmentation de la réponse immunitaire induite.

Dans la présente invention, l'optimisation de l'antigène d'intérêt consiste de préférence en la délétion du fragment de la séquence nucléotidique codant pour le domaine transmembranaire de l'antigène d'intérêt (par délétion, on entend la délétion totale ou une délétion partielle suffisante pour que le domaine transmembranaire ne soit plus, ou substantiellement plus, fonctionnel), et/ou en l'addition en phase d'une séquence nucléotidique codant pour le signal tPA (Montgomery *et al.* Cell. Mol. Biol. 1997. **43.** 285-292 ; Harris *et al.* Mol. Biol. Med 1986. **3.** 279-292), et/ou en l'insertion d'intron stabilisateur en amont du gène à exprimer. La délétion du fragment d'ADN codant pour le domaine transmembranaire de l'antigène d'intérêt favorise la sécrétion vers le milieu extracellulaire des antigènes ainsi tronqués et ainsi augmente leurs possibilités de contact avec les cellules du système immunitaire. L'insertion de la séquence nucléotidique codant pour le signal tPA facilite la traductibilité de l'ARN messager auquel le signal tPA est joint, et augmente ainsi le niveau d'expression de cet ARN messager et donc la production d'antigènes. Le signal tPA joue aussi un rôle dans la sécrétion de l'antigène synthétisé.

D'autres séquences nucléotidiques codant pour des peptides signaux peuvent être utilisées, notamment celles du peptide signal de la mélittine provenant des abeilles (Sisk W. P. *et al.,* 1994, J. Virol., **68**, 766-775).

L'insertion d'un intron stabilisateur dans le gène codant pour l'antigène d'intérêt évite les épissages aberrants de son ARN messager et maintient l'intégrité physique de ce dernier.

De préférence le signal tPA est d'origine humaine. La séquence nucléotidique du signal tPA humain est accessible auprès de la base de données GenBank sous le numéro d'accès NM_000930. De préférence, l'intron est l'intron II du gène de la bêta-globine du lapin (van Ooyen *et al.* Science 1979. **206.** 337-344), dont la séquence nucléotidique est accessible auprès de la base de données GenBank sous le numéro d'accès V00882 et référencée sous intron n°2.

La présente invention a pour objet un vaccin ADN amélioré capable d'induire une réponse immunitaire efficace et protectrice chez les bovins contre la rhinotrachéite infectieuse bovine (IBR).

Le virus responsable de la rhinotrachéite infectieuse bovine est un virus herpès bovin de type 1 (BHV-1), membre de la famille des *Alphaherpesvirinae* (Babiuk L A. *et al.* 1996. Vet. Microbiol. **53.** 31-42). Des séquences nucléotidiques codant pour les glycoprotéines gB, gC et gD, sont connues et accessibles auprès de la base de données GenBank sous le numéro d'accès AJO04801.

Selon l'invention, le vaccin ADN contre IBR est amélioré par sa formulation avec un adjuvant le DMRIE-DOPE. Facultativement, cela peut être combiné avec soit l'addition de GM-CSF bovin (Maliszewski *et al.* Molec. Immunol. 1988. **25.** 843-850), soit l'optimisation d'au moins un antigène de IBR, enfin soit l'addition de GM-CSF bovin et l'optimisation d'au moins un antigène de IBR.

Une séquence nucléotidique codant pour le GM-CSF bovin est accessible auprès de la base de données GenBank sous le numéro d'accès U22385.

L'addition de GM-CSF bovin peut se faire par l'incorporation du polypeptide GM-CSF bovin dans la composition vaccinale ou de préférence par l'insertion de la séquence nucléotidique codant pour le GM-CSF bovin dans un vecteur d'expression *in vivo,* de préférence un plasmide. De préférence, la séquence nucléotidique codant pour le GM-CSF bovin est insérée dans un deuxième plasmide d'expression (e.g. pLF1032 exemple 13), différent de celui (ou de ceux) dans lequel est inséré le ou les gènes codant pour le ou les antigènes de IBR.

L'optimisation des antigènes issus de IBR se fait par substitution, par une séquence "signal", notamment celle du signal tPA d'origine humaine (GenBank numéro d'accès NM_000930), de la séquence du peptide signal de la glycoprotéine gB et/ou de la glycoprotéine gC et/ou de la glycoprotéine gD, et/ou par la délétion du fragment d'ADN codant pour le domaine transmembranaire de gB et/ou de gC et/ou de gD. La délétion du fragment d'ADN codant pour le domaine transmembranaire d'une de ces glycoprotéines s'accompagne de préférence de la partie C terminale contiguë (portion cytoplasmique de la glycoprotéine). Le vaccin ADN contre IBR selon l'invention peut donc coder et exprimer un seul antigène d'IBR optimisé (gB, gC ou gD) ou deux d'entre eux ou les trois, c'est-à-dire gB optimisé, gC optimisé et gD optimisé.

Des séquences nucléotidiques codant pour les antigènes de BHV-1 utilisables dans la présente invention et différentes constructions de vecteurs d'expression sont données dans les exemples annexés et dans FR-A1-2751229, notamment dans les exemples 7 et 8, et dans les figures 3 et 4.

De manière préférentielle selon l'invention, le vaccin ADN contre BHV-1. est formulé avec du DMRIE-DOPE, et est composé d'un plasmide d'expression (e.g. pPB281, exemple 3.1.2) codant pour l'antigène gB de BHV-1 optimisé par la délétion du fragment de la séquence nucléotidique codant pour le domaine transmembranaire et la partie C terminale contiguë, d'un deuxième plasmide d'expression (e.g. pPB292, exemple 3.2.2) codant pour l'antigène gC de BHV-1 optimisé par la délétion du fragment de la séquence nucléotidique codant pour le domaine transmembranaire et la partie C terminale contiguë, et d'un troisième plasmide d'expression (e.g. pPB284, exemple 3.3.2) codant pour l'antigène gD de BHV-1 optimisé par la délétion du fragment de la séquence nucléotidique codant pour le domaine transmembranaire et la partie C terminale contiguë.

De manière générale, et pas seulement pour BHV-1, la partie C terminale contiguë à la séquence codant pour le domaine transmembranaire peut être conservée, Il est toutefois souvent plus aisé de la déléter en même temps que la séquence codant pour le domaine transmembranaire.

Les vaccins ADN améliorés selon l'invention peuvent être également associés avec au moins un vaccin classique (inactivé, vivant atténué, sous-unités) ou vaccin recombiné utilisant un vecteur d'expression *in vivo* (e.g. poxvirus, adénovirus, herpèsvirus) dirigé contre au moins un pathogène différent infectant ta même espèce, ou être utilisés comme rappels de tels vaccins.

L'homme de l'art peut se reporter à FR-A1-2751229 pour les méthodes pour construire les plasmides contenant ces valences bovines.

Est également décrite l'utilisation d'une ou plusieurs doses du vaccin ADN amélioré chez la femelle gestante en vue du transfert passif de l'immunité ou le jeune animal ou l'adulte.

La quantité d'ADN utilisée dans les vaccins selon la présente Invention est comprise entre environ 10 µg et environ 1000 µg, et Préférentiellement entre environ 50 µg et environ 500 µg, pour un plasmide donné. L'homme de l'art possède les compétences nécessaires pour définir précisément la dose efficace d'ADN à utiliser pour chaque protocole de vaccination.

Les volumes de dose peuvent être de préférence compris entre 0,2 et 5 ml, de préférence entre 1 et 3 ml.

Les vaccins ADN améliorés selon l'invention peuvent être administrés, par les différentes voles d'administration proposées dans l'art antérieur pour la vaccination polynucléotidique et au moyen des techniques d'administration connues.

Suivant une modalité préférée de l'invention, l'administration est faite par la voie intramusculaire, sous-cutanée ou à l'aide d'un injecteur sans aiguille par voie intradermique des vaccins ADN améliorés selon l'invention.

L'invention va être maintenant décrite plus en détail à l'aide de modes de réalisation pris à titre d'exemples non limitatifs et se référant aux dessins dans lesquels :
Figure N° 1 : Plasmide pVR1012
Figure N° 2 : Plasmide pAB110

### Listes des séquences :

SEQ ID NO 1 : oligonucléotide PB326
SEQ ID NO 2 : oligonucléotide PB329
SEQ ID NO 3 : oligonucléotide SB090
SEQ ID NO 4 : oligonucléotide SB091
SEQ ID NO 5 : oligonucléotide LF001
SEQ ID NO 6 : oligonucléotide LF002
SEQ ID NO 7 : oligonucléotide PB234
SEQ ID NO 8 : oligonucléotide PB235
SEQ ID NO 9 : oligonucléotide PB511
SEQ ID NO 10 : oligonucléotide PB512
SEQ ID NO 11 : oligonucléotide SB221
SEQ ID NO 12 : oligonucléotide SB222
SEQ ID NO 13 : oligonucléotide PB507
SEQ ID NO 14: oligonucléotide PB508
SEQ ID NO 15 : oligonucléotide PB513
SEQ ID NO 16 : oligonucléotide PB514
SEQ ID NO 17 : oligonucléotide SB223
SEO ID NO 18 : oligonucléotide SB224
SEQ ID NO 19 : oligonucléotide PB497
SEQ ID NO 20 : oligonucléotide PB498
SEQ ID NO 21 : oligonucléotide SB225
SEQ ID NO 22 : oligonucléotide SB226
SEQ ID NO 103 : oligonucléotide LF054
SEQ ID NO 104 : oligonucléotide LF055

### EXEMPLES :

Chaque gène BHV-1 codant pour les principaux antigènes (forme native et forme modifiée) fait l'objet d'une construction particulière dans un plasmide d'expression eucaryote. Les formes secrétées des antigènes sont obtenues par délétion des fragments de gènes codant pour les domaines trans-membranaires et cytoplasmiques. Dans tous les cas, les domaines trans-membranaires des protéines sont identifiés sur la base des profils d'hydropathie (sur MacVector 6.5) des séquences protéiques correspondantes.

### Exemple 1 : Méthodes de biologie moléculaire

### 1.1 Extraction d'ADN génomique viral

Des suspensions virales sont traitées par la protéinase K (100 mg/ml final) en présence de sodium dodécyl sulfate (SDS) (0.5% final) pendant 2 heures à 37 °C. L'ADN viral est ensuite extrait à l'aide d'un mélange phénot/chtorophorme, puis précipité avec deux volumes d'éthanol absolu à -20°C pendant 16 heures et ensuite centrifugé à 10 000 g pendant 15 minutes à 4°C. Les culots d'ADN sont séchés, puis repris dans un volume minimum d'eau ultrapure stérile.

### 1.2 Isolement d'ARN génomique viral

L'ARN génomique de chaque virus est extrait en utilisant la technique du " thiocyanate de guanidinium/phénol-chlorophorme " décrite par P. Chomczynski et N. Sacchi (Anal. Biochem. 1987. **162**. 156-159).

### 1.3 Techniques de biologie moléculaire

Toutes les constructions de plasmides sont réalisées en utilisant les techniques standard de biologie moléculaire décrites par Sambrook et al. (Molecular Cloning : A Laboratory Manual. 2^{nd} Edition . Cold Spring Harbor Laboratory, Cold Spring Harbor, New York, 1989). Tous les fragments de restriction utilisés pour la présente invention sont isolés à l'aide du kit " Geneclean " (BIO101 Inc., La Jolla, CA). Pour toutes les constructions, les fragments d'ADN clonés, ainsi que les jonctions avec le vecteur d'expression, sont séquencés par la méthode de Sanger (Sambrook *et al.* 1989).

### 1.4 PCR et RT-PCR

Les oligonucléotides spécifiques des gènes ou des fragments de gènes clonés sont synthétisés, certains d'entre eux comportant dans certains cas à leur extrémité 5' des sites de restriction facilitant le clonage des fragments amplifiés. Les réactions de transription inverse (RT) et l'amplification en chaîne par polymérase (PCR) sont effectuées selon des techniques standard (Sambrook *et al.* 1989).

### 1.5 Purification de plasmides à grande échelle

La production, à l'échelle de la dizaine de mg, de plasmides purifiés entrant dans les compositions vaccinales est effectuée par la méthode des gradients de chlorure de césium-bromure d'éthidium (Sambrook *et al.* 1989).

### Exemple 2 : Constructions plasmidiques de base

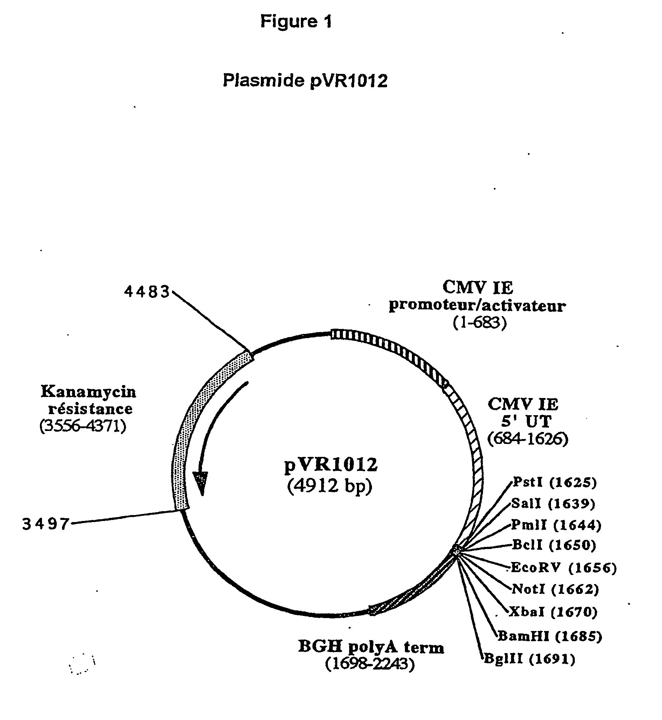

Le plasmide d'expression eucaryote pVR1020 (C. J. Luke *et al.* J. of Infectious Diseases. 1997. 175. 95-97), dérivé du plasmide pVR1012 (Figure N° 1, figure 1 et exemple 7 de WO-A-9803199), contient la phase codante de la séquence-signal de l'activateur du plasminogène tissulaire humain (tPA).

Un plasmide pVR1020 est modifié par digestion BamHI-BgIII et insertion d'une séquence contenant plusieurs sites de clonage (BamHI, NotI, EcoRI, XbaI, PmII, PstI, BgIII) et résultant de l'appartement des oligonucléotides suivants :
PB326 (40 mer) (SEQ ID NO 1)
   5' GATCTGCAGCACGTGTCTAGAGGATATCGAATTCGCGGCC 3' et
PB329 (40 mer) (SEQ ID NO 2)

### 5' GATCCGCGGCCGCGAATTCGATATCCTCTAGACACGTGCT 3'.

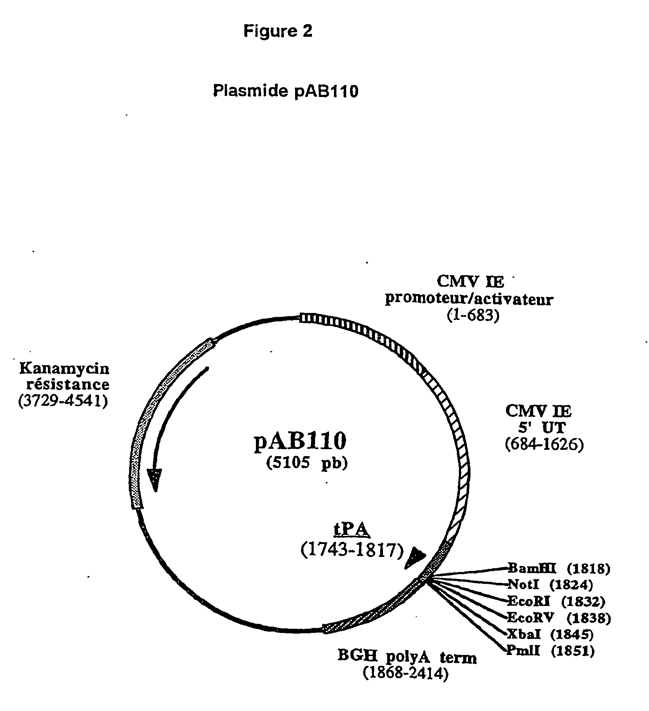

Le vecteur ainsi obtenu, d'une taille d'environ 5105 paires de bases (ou pb), est nommé pAB110 (Figure N° 2).

L'intron Il du gène de la β-globine de lapin est cloné dans le vecteur pCRII (Invitrogen, Carlsbad, CA, USA) après obtention du fragment d'ADN correspondant par PCR à l'aide des oligonucléotides suivants :
SB090 (20 mer) (SEQ ID NO 3)
   5' TTGGGGACCCTTGATTGTTC 3' et
SB091 (21 mer) (SEQ ID NO 4)
   5' CTGTAGGAAAAAGAAGAAGGC 3'
en utilisant comme matrice l'ADN génomique de cellules du sang périphérique de lapin. Le plasmide résultant est désigné pNS050.

Le plasmide d'expression pAB110 est modifié par l'introduction de la séquence de l'intron II du gène de la globine du lapin dans le site Sall situé en amont de l'ATG du peptide signal de l'activateur du plasminogène tissulaire (tPA). La séquence de l'intron II du gène de la globine du lapin est amplifiée par amplification en chaîne par polymérase (ACP ou PCR) à partir du plasmide pNS050 en utilisant le couple d'oligonucléotides suivants :
LF001 (30 mer) (SEQ ID NO 5)
   5' CTCCATGTCGACTTGGGGACCCTTGATTGT 3' et
LF002 (30 mer) (SEQ ID NO 6)
   5' CTCCATGTCGACCTGTAGGAAAAAGAAGAA 3'

Le produit de PCR (573 paires de bases ou pb) est digéré par SalI et cloné dans le plasmide pAB110 préalablement linéarisé par SalI, pour générer le plasmide pLF999 d'environ 5678 pb.

### Exemple 3 : Plasmides codant pour les différentes formes des antigènes du virus herpès bovin de type 1 (BHV-1)

Des fragments d'ADN viral contenant les gènes gB, gC et gD de la souche B901 de BHV-1 sont isolés en digérant le génome viral par différents enzymes de restriction, en les séparant par électrophorèse en gel d'agarose et en les analysant par Southem blot à l'aide de sondes correspondant à des fragments des gènes gB, gC et gD de la souche ST de BHV-1 (Leung-Tack P. *et al.* Virology. 1994. 199. 409-421). La souche BHV-1 Colorado [Cooper] (ATCC numéro VR-864) peut également être utilisée. Les fragments ainsi identifiés sont clonés dans le vecteur pBluescript SK+ (Stratagene, La Jolla, CA, USA) et sont à l'origine des clonages des trois gènes dans le vecteur d'expression pVR1012.

### 3.1 Plasmides codant pour les différentes formes de BHV-1 gB

### 3.1.1 pPB280 : gène gB (forme native) cloné dans le vecteur pVR1012

Deux fragments Xhol-Xhol contenant les parties 5' et 3' du gène gB de BHV-1 sont identifiés par Southem blot et clonés dans le vecteur pBluescript SK+ (Stratagene, La Jolla, CA, USA) préalablement digéré par XhoI. Les plasmides ainsi obtenus sont respectivement désignés pPB128 et pPB117.

Le plasmide pPB128, contenant le fragment 5' du gène gB, est digéré par NotI et Xhol, générant un fragment de 1708 pb (fragment A).

Le plasmide pPB117, contenant la partie 3' du gène gB, est digéré par XhoI et StuI, générant un fragment de 1345 pb. Ce dernier fragment est cloné dans le vecteur pBluescript KS+ (Stratagene, La Jolla, CA, USA) préalablement digéré par EcoRV et Xhol. Le plasmide résultant est nommé pPB279. Le plasmide pPB279 est ensuite digéré par XhoI et BamHl, générant un fragment d'ADN de 1413 pb (fragment B).

Les fragments A et B sont ensuite clonés dans un vecteur pBluescript KS+ digéré par Notl et BamHl, générant le plasmide pPB278 (environ 6063 pb) et permettant la reconstitution du gène gB de BHV-1.

Le vecteur pPB278 sert ensuite de matrice lors d'une réaction de PCR réalisée avec les oligonucléotides suivants :
PB234 (30 mer) (SEQ ID NO 7)
   5' TTGTCGACATGGCCGCTCGCGGCGGTGCTG 3' et
PB235 (21 mer) (SEQ ID NO 8)
   5' GCAGGGCAGCGGCTAGCGCGG 3'.

Le produit de PCR (146 pb) est ensuite digéré par les enzymes de restriction SalI et NheI.

Le plasmide pPB278 est digéré par Nhel et BarnHI. Le fragment de 2728 pb ainsi obtenu et le fragment PCR précédemment digéré sont ligaturés dans le vecteur pVR1012 (exemple 2) préalablement digéré par Sali et BamHI, générant ainsi le plasmide pPB280, d'une taille d'environ 7742 pb.

Le gène gB de BHV-1 code pour une protéine de 933 acides aminés.

### 3.1.2 pPB281: gène gB (forme Δ[TM-Cter]) cloné dans le vecteur pVR1012

La forme tronquée (délétée de ses domaines transmembranaire (TM) et carboxy-terminal (Cter)) du gène gB de BHV-1 est obtenue en ligaturant dans le plasmide pVR1012 (exemple 2) prédigéré par SalI et BamHI, à la fois un fragment d'une taille de 2234 pb obtenu après digestion par SalI-PvuII du plasmide pPB280 (exemple 3.1.1) et un fragment de 56 pb obtenu par l'appariement des oligonucléotides suivants :
PB511 (52 mer) (SEQ ID NO 9)
   5'GTGCACGAGCTCCGGTTCTACGACATTGACCGCGTGGTCAAGACGGACT GAG 3' et
PB512 (57 mer) (SEQ ID NO 10)
   5'GATCCTCAGTCCGTCTTGACCACGCGGTCAATGTCGTAGAACCGGAGCTC GTGCAG 3'.

Le plasmide ainsi généré a une taille d'environ 7154 pb et est nommé pPB281. Le gène gB tronqué de BHV-1 code pour une protéine de 759 acides aminés.

### 3.1.3 pSB115 : gène gB (forme tPA Δ[TM-Cter]) cloné dans le vecteur pAB110

La forme tPA Δ[TM-Cter] du gène gB de BHV-1 est amplifiée par PCR à partir de la matrice pPB281 (exemple 3.1.2) et à l'aide des amorces suivantes :
SB221 (39 mer) (SEQ ID NO 11)
   5' AAAATTTCGATATCCGCCGCGGGGCGACCGGCGACAACG 3' et
SB222 (33 mer) (SEQ ID NO 12)
   5' GGAAGATCTTCAGTCCGTCTTGACCACGCGGTC 3'.

Le produit d'amplification (2088 pb) est digéré par les enzymes EcoRV et BglII et cloné dans le vecteur pAB110 (exemple 2) préalablement digéré par EcoRV et BglII, générant le plasmide pSB115, d'une taille d'environ 7154 pb.

La forme tPA Δ[TM-Cter] du gène gB code pour une glycoprotéine de 729 acides aminés, contenant le domaine extracellulaire de la glycoprotéine gB de BHV-1.

### 3.2. Plasmides codant pour les différentes formes de BHV-1 gC

### 3.2.1 pPB264 : gène gC (forme native) cloné dans le vecteur pVR1012

Un fragment BamHI-HindIII de 3,5 kb contenant le gène complet gC de BHV-1 est identifié par Southern blot et cloné dans le vecteur pBluescript SK+. Le plasmide ainsi obtenu est nommé pPB287.

Le plasmide pPB287 est ensuite digéré par Ncol-BssSl. Un fragment de digestion d'une taille de 1492 pb est obtenu. Il est ligaturé avec un fragment d'ADN de synthèse obtenu par l'appariement des oligonucléotides suivants :
PB507 (37 mer) (SEQ ID NO 13)
   5' TCGTGCCTGCGGCGCAAGGCCCGGGCGCGCCTGTAGT 3' et
PB508 (37 mer) (SEQ ID NO 14)
   5' CTAGACTACAGGCGCGCCCGGGCCTTGCGCCGCAGGC 3',
dans le plasmide pLitmus 28 (New England Biolabs, Inc., Beverly, MA, USA) prédigéré par Ncol et Xbal, générant le plasmide intermédiaire pPB290.

Le fragment de 1554 pb issu de la digestion de pPB290 par PstI et XbaI est cloné dans le vecteur pVR1012 (exemple 2) préalablement digéré par Pstl et XbaI, générant ainsi le plasmide pPB264, d'une taille d'environ 6427 pb. Le gène gC de BHV-1 code pour une protéine de 508 acides aminés.

### 3.2.2 pPB292 : gène gC (forme Δ[TM-Cter]) cloné dans le vecteur pVR1012

La forme tronquée du gène gC de BHV-1 est obtenue en ligaturant les trois fragments d'ADN suivants dans le vecteur pVR1012 (exemple 2) préalablement digéré par Pstl et Xbal :
(a) un fragment de 1035 pb issu de la digestion de pPB264 (exemple 3.2.1) par Pstl et Xhol,
(b) un fragment de 350 pb issu de la digestion par Xhol et BanI de pPB264 et
(c) un fragment de synthèse de 43 pb résultant de l'appariement des oligonucléotides PB513 et PB514.

Ces oligonucléotides sont les suivants :
PB513 (43 mer) (SEQ ID NO 15)
   5' GCACCGCTGCCCGAGTTCTCCGCGACCGCCACGTACGACTAGT 3' et
PB514 (43 mer) (SEQ ID NO 16)
   5' CTAGACTAGTCGTACGTGGCGGTCGCGGAGAACTCGGGCAGCG 3'.

Le plasmide d'une taille d'environ 6305 pb, ainsi obtenu, est nommé pPB292. Le gène gC tronqué de BHV-1 code pour une protéine de 466 acides aminés.

### 3.2.3 pSB116 : gène gC (forme tPA Δ[TM-Cter]) cloné dans le vecteur pAB110

La forme tPA Δ[TM-Cter] du gène gC de BHV-1 est amplifiée par PCR à partir de la matrice pPB292 (exemple 3.2.2) et à l'aide des amorces suivantes :
SB223 (39 mer) (SEQ ID NO 17)
   5'AAAATTTCGATATCCCGGCGGGGGCTCGCCGAGGAGGCG 3' et
SB224 (32 mer) (SEQ ID NO 18)
   5' GGAAGATCTCTAGTCGTACGTGGCGGTCGCGG 3'.

Le produit d'amplification (1362 pb) est digéré par les enzymes EcoRV et BgIII et cloné dans le vecteur pAB110 (exemple 2) préalablement digéré par EcoRV et BgIII, générant le plasmide pSB116, d'une taille d'environ 6404 pb.

La forme tPA Δ[TM-Cter] du gène gC code pour une glycoprotéine de 479 acides aminés, contenant le domaine extracellulaire de la glycoprotéine gC de BHV-1.

### 3.3 Plasmides codant pour les différentes formes de BHV-1 gD

### 3.3.1 pPB148 : gène gD (forme native) cloné dans le vecteur pVR1012

Un fragment Xhol-Xhol de 5 kb contenant le gène gD de BHV-1 est identifié par Southem blot et cloné dans le vecteur pBluescript SK+ prédigéré par Xhol, générant le plasmide pPB147.

Un fragment de 325 pb issu de la digestion de pPB147 par Ndel et BsrBl et un fragment de 943 pb isssu de la digestion de pPB147 par Ndel et Styl sont ensuite ligaturés dans le vecteur pVR1012 (exemple 2) prédigéré par EcoRV et Xbal, générant ainsi le plasmide pPB148, d'une taille d'environ 6171 pb. Le gène gD de BHV-1 code pour une protéine de 417 acides aminés.

### 3.3.2 pPB284 : gène gD (forme Δ[TM-Cter]) cloné dans le vecteur pVR1012

Le gène gD tronqué de BHV-1 est obtenu à partir d'un fragment obtenu après amplification par PCR réalisée sur l'ADN génomique de la souche B901 du virus BHV-1 préalablement digéré par PstI et Xbal et à l'aide du couple d'amorces suivantes :
PB497 (33 mer) (SEQ ID NO 19)
   5' TTTCTGCAGATGCAAGGGCCGACATTGGCCGTG 3' et
PB498 (31 mer) (SEQ ID NO 20)
   5' TTTCTAGATTAGGGCGTAGCGGGGGCGGGCG 3'.

Ce fragment de PCR est ensuite cloné dans le plasmide pVR1012 (exemple 2) préalablement digéré par PstI et Xbal, générant le plasmide pPB284, d'une taille d'environ 5943 pb. Le gène gD tronqué de BHV-1 code pour une protéine de 355 acides aminés.

### 3.3.3 pSB117 : gène gD (forme tPA Δ[TM-Cter]) cloné dans le vecteur pAB110

La forme tPA Δ[TM-Cter] du gène gD de BHV-1 est amplifiée par PCR à partir de la matrice pPB284 (exemple 3.3.2) et à l'aide des amorces suivantes :
SB225 (39 mer) (SEQ ID NO 21)
   5' AAAATTTCGATATCCCCCGCGCCGCGGGTGACGGTATAC 3' et
SB226 (33 mer) (SEQ ID NO 22)
   5' GGAAGATCTTTAGGGCGTAGCGGGGGCGGGCGG 3'.

Le produit d'amplification (1029 pb) est digéré par les enzymes EcoRV et BglII et cloné dans le vecteur pAB110 (exemple 2) préalablement digéré par EcoRV et BglII, générant le plasmide pSB117, d'une taille d'environ 6071 pb.

La forme tPA Δ[TM-Cter] du gène gD code pour une glycoprotéine de 368 acides aminés, contenant le domaine extracellulaire de la glycoprotéine gD de BHV-1.

### Exemple 4: Plasmide codant pour le GM-CSF bovin

L'ADNc du gène GM-CSF bovin est synthétisé à partir de d'ARN cellulaire de cellules mononuclées sanguines bovines à l'aide de l'amorce LF065 et amplifié par une réaction de PCR à l'aide du couple d'oligonucléotides suivants :
LF054 (36 mer) (SEQ ID NO 103)
   5' CATATCGTCGACATGTGGCTGCAGAACCTGCTTCTC 3' et
LF055 (34 mer) (SEQ ID NO 104)
   5' CATGACCAGATCTTCACTTCTGGGCTGGTTCCCA 3'.

Le fragment d'ADN de 437 pb obtenu par digestion du produit de PCR par SalI et BgIII est ligaturé avec un fragment de 4860 pb résultant de la digestion de pVB1012 (exemple 2) par SalI et BgIII pour générer le plasmide pLF1032 (environ 5297 pb). Le gène GM-CSF bovin code pour une protéine de 143 acides aminés.

### Exemple 5: Formulation des plasmides vaccinaux

La solution d'ADN contenant un ou plusieurs plasmides selon les exemples 3 et 4 est concentrée par précipitation éthanolique comme décrit dans Sambrook *et al.* (1989). Le culot d'ADN est repris par une solution de NaCl 0.9% de façon à obtenir une concentration de 1 mg/ml. Une solution de DMRIE-DOPE à 0,75mM est préparée par reprise d'un lyophilisat de DMRIE-DOPE par un volume adapté d'H₂O stérile.

La formation des complexes ADN plasmidique-lipide est réalisée par dilution à parties égales de la solution de DMRIE-DOPE 0.75 mM par la solution d'ADN à 1 mg/ml dans NaCl 0.9%. La solution d'ADN est introduite progressivement à l'aide d'une aiguille sertie 26G le long de la paroi du flacon contenant la solution de lipide cationique de façon à éviter la formation de mousse. On procède à une agitation douce dès que les deux solutions sont mélangées. On obtient en final une composition comprenant 0,375 mM de DMRIE-DOPE et 500 µg/ml de plasmide.

Il est souhaitable que l'ensemble des solutions utilisées soient à température ambiante pour l'ensemble des opérations décrites ci-dessus. On laisse la complexation ADN/DMRIE-DOPE se mettre en place à température ambiante pendant 30 minutes environ avant de procéder à l'immunisation des animaux.

### Exemple 6: Immunisation des bovins contre BHV-1

12 bovins sont randomisés en 3 groupes de 4 animaux.

Le groupe 1 constitue le groupe des animaux témoins.

On administre aux animaux du groupe 2 un mélange de plasmides vaccinaux pPB281 (codant pour gB de BHV-1 sous une forme Δ[TM-Cter], exemple 3.1.2), pPB292 (codant pour gC de BHV-1 sous une forme Δ[TM-Cter], exemple 3.2.2) et pPB284 (codant pour gD de BHV-1 sous une forme Δ[TM-Cter], exemple 3.3.2).

On administre aux animaux du groupe 3 le même mélange que celui du groupe 2 mais formulé avec du DMRIE-DOPE comme cela est décrit dans l'exemple 5.

Une injection de 10 ml par voie intramusculaire est pratiquée à l'aide de seringues munies d'aiguille sur chaque bovin, et est répétée 21 jours plus tard. La masse totale de chaque plasmide utilisée lors de chaque immunisation est de 1500 µg.

L'homme de l'art possède les connaissances nécessaires pour adapter le volume ou la concentration en fonction de la dose de plasmide requise.

Un suivi de la réponse sérologique induite par les deux mélanges de plasmides vaccins exprimant les antigènes gB, gC et gD de BHV-1 est réalisé sur une période de 35 jours après la primovaccination.

Les résultats sont présentés dans le tableau qui suit :

| Plasmides | Formulation | Antigènes | Dose | SN à J28 | SN à J35 |
|---|---|---|---|---|---|
| contrôle | --- | --- | --- | 0.2+/-0,0 | 0,2+/-0,0 |
| pPB281 | --- | gB Δ[TM-Cter] | 1500 µg | 1,0+/0,5 | 1,2+/-0,8 |
| pPB292 | | gC Δ[TM-Cter] | 1500 µg | | |
| pPB294 | | gD Δ[TM-Cter] | 1500 µg | | |
| pPB281 | DMRIE-DOPE | gB Δ[TM-Cter] | 1500 µg | 2,1+/0,6 | 2,7+/-0,6 |
| pPB292 | | gC Δ[TM-Cter] | 1500 µg | | |
| pPB294 | | gD Δ[TM-Cter] | 1500 µg | | |

## Revendications

1. Vaccin ADN comprenant un plasmide contenant un acide nucléique codant pour un immunogène du virus herpès bovin de type 1 (BHV-1) et les éléments nécessaires à son expression *in vivo*, du N-(2-hydroxyéthyl)-N,N-(diméthyl-2,3-bis(tétradécyloxy)-1-propanammonimum (DMRIE) et de la dioléoyl-phosphortidyl-éthanolamine (DOPE).

2. Vaccin selon la revendication 1, comprenant en outre une protéine granulocyte-macrophage colony stimulating factor (GM-CSF).

3. Vaccin selon l'une quelconque des revendications 1 ou 2, comprenant en outre un vecteur d'expression contenant le gène codant pour la protéine GM-CSF bovine, dans des conditions permettant l'expression in vivo de ce gène.

4. Vaccin selon la revendication 3, dans lequel le vecteur d'expression est un plasmide.

5. Vaccin selon l'une quelconque des revendications 1 à 4, dans lequel le plasmide contient un acide nucléique codant pour un immunogène du BHV-1, acide nucléique dont on a délété la partie codant pour le domaine transmembranaire.

6. Vaccin selon l'une quelconque des revendications 1 à 5, dans lequel le plasmide contient un acide nucléique codant pour un immunogène du BHV-1, et une séquence signal hétérologue, de préférence celle de l'activateur de plasminogène tissulaire humain (tPA).

7. Vaccin selon l'une quelconque des revendications 1 à 6, dans lequel le plasmide contient un acide nucléique comportant la séquence nucléique codant pour un immunogène du BHV-1 et un intron stabilisateur.

8. Vaccin selon la revendication 7, dans lequel l'intron est l'intron II du gène de la bêta-globine du lapin.

9. Vaccin selon l'une quelconque des revendications 1 à 8, comprenant un plasmide contenant un acide nucléique codant pour la glycoprotéine gB de BHV-1.

10. Vaccin selon l'une quelconque des revendications 1 à 8, comprenant un plasmide contenant un acide nucléique codant pour la glycoprotéine gC de BHV-1.

11. Vaccin selon l'une des revendications 1 à 8, comprenant un plasmide contenant un acide nucléique codant pour la glycoprotéine gD de BHV-1.

12. Vaccin selon la revendication 9, dans lequel la séquence du gène gB comprend une séquence signal hétérologue, notamment celle du signal tPA d'origine humaine, à la place de la séquence du peptide signal de la glycoprotéine gB, et/ou la délétion de la partie du gène gB codant pour le domaine transmembranaire.

13. Vaccin selon la revendication 10, dans lequel la séquence du gène gC comprend une séquence signal hétérologue, notamment celle du signal tPA d'origine humaine, à la place de la séquence signal de la glycoprotéine gC, et/ou la délétion de la partie du gène gC codant pour le domaine transmembranaire.

14. Vaccin selon la revendication 11, dans lequel la séquence du gène gD comprend une séquence signal hétérologue, notamment celle du signal tPA d'origine humaine, à la place de la séquence du peptide signal de la glyocoprotéine gD, et/ou la délétion de la partie du gène gD codant pour le domaine transmembranaire.

15. Vaccin selon la revendication 1, comprenant un plasmide d'expression comprenant un acide nucléique codant pour gB de BHV-1 délété du fragment codant pour le domaine transmembranaire et la partie C terminale contiguë, et un deuxième plasmide d'expression comprenant un acide nucléique codant pour gD de BHV-1 délété du fragment codant pour le domaine transmembranaire et la partie C terminale contiguë.

## Claims

1. A DNA vaccine comprising a plasmid containing a nucleic acid coding for an immunogen of bovine herpes virus type 1 (BHV-1) and the elements necessary for its in vivo expression, N-(2-hydroxyethyl)-N,N-dimethyl-2,3-bis(tetradecyloxy)-1-propanammonium (DMRIE) and dioleoyl-phosphatidyl-ethanolamine (DOPE).

2. A vaccine according to claim 1, further comprising a granulocyte-macrophage colony stimulating factor (GM-CSF) protein.

3. A vaccine according to claim 1 or claim 2, further comprising an expression vector containing the gene coding for the bovine GM-CSF protein under conditions allowing in vivo expression of said gene.

4. A vaccine according to claim 3, in which the expression vector is a plasmid.

5. A vaccine according to any one of claims I to 4, in which the plasmid contains a nucleic acid coding for a BHV-1 immunogen, from which nucleic acid the portion coding for the transmembrane domain has been deleted.

6. A vaccine according to any one of claims 1 to 5, in which the the plasmid contains a nucleic acid coding for a BHV-1 immunogen, and a heterologous signal sequence, preferably that of the human tissue plasminogen activator (tPA).

7. A vaccine according to any one of claims 1 to 6, in which the plasmid contains a nucleic acid comprising the nucleic sequence coding for a BHV-1 immunogen and a stabilizing intron.

8. A vaccine according to claim 7, in which the intron is the intron II of the rabbit beta-globin gene.

9. A vaccine according to any one of claims 1 to 8, comprising a plasmid containing a nucleic acid coding for the gB glycoprotein of BHV-1.

10. A vaccine according to any one of claims 1 to 8, comprising a plasmid containing a nucleic acid coding for the gC glycoprotein of BHV-1.

11. A vaccine according to any one of claims 1 to 8, comprising a plasmid containing a nucleic acid coding for the gD glycoprotein of BHV-1.

12. A vaccine according to claim 9, in which the sequence for the gB gene comprises a heterologous signal sequence, in particular that of the human tPA signal, instead of the sequence for the signal peptide of the gB glycoprotein, and/or a deletion of the portion of the gB gene coding for the transmembrane domain.

13. A vaccine according to claim 10, in which the sequence for the gC gene comprises a heterologous signal sequence, in particular that of the human tPA signal, instead of the sequence for the signal peptide of the gC glycoprotein, and/or a deletion of the portion of the gC gene coding for the transmembrane domain.

14. A vaccine according to claim 10, in which the sequence for the gD gene comprises a heterologous signal sequence, in particular that of the human tPA signal, instead of the sequence for the signal peptide of the gD glycoprotein, and/or a deletion of the portion of the gD gene coding for the transmembrane domain.

15. A vaccine according to claim 1, comprising an expression plasmid comprising a nucleic acid coding for the gB of BHV-1 with a deletion of the fragment coding for the transmembrane domain and the contiguous C-terminal portion, and a second expression plasmid comprising a nucleic acid coding for the gD of BHV-1 with a deletion of the fragment coding for the transmembrane domain and the contiguous C-terminal portion.

## Patentansprüche

1. DNA-Impfstoff, umfassend ein Plasmid, das eine Nucleinsäure enthält, die ein Immunogen des bovinen Herpesvirus Typ 1 (BHV-1) codiert, sowie die Elemente, die zu seiner Expression *in vivo* erforderlich sind, N-(2-Hydroxyethyl)-N, N-(Dimethyl-2,3-bis(Tetradecyloxy)-1-Propanammonium (DMRIE) und Dioleoylphosphatidylethanolamin (DOPE).

2. Impfstoff gemäß Anspruch 1, der zusätzlich ein Granulozyten-Makrophagen-Kolonie-stimulierendes Faktor-Protein (GM-CSF) umfasst.

3. Impfstoff gemäß einem der Ansprüche 1 oder 2, der zusätzlich einen Expressionsvektor umfasst, der das das bovine GM-CSF-Protein codierende Gen enthält, unter Bedingungen, die die *in vivo*-Expression dieses Gens erlauben.

4. Impfstoff gemäß Anspruch 3, wobei der Expressionsvektor ein Plasmid ist.

5. Impfstoff gemäß einem der Ansprüche 1 bis 4, in dem das Plasmid eine ein Immunogen von BHV-1 codierende Nucleinsäure umfasst, wobei der den transmembranen Bereich codierende Teil der Nucleinsäure deletiert wurde.

6. Impfstoff gemäß einem der Ansprüche 1 bis 5, wobei das Plasmid eine ein Immunogen von BHV-1 codierende Nucleinsäure sowie eine heterologe Signalsequenz enthält, vorzugsweise die Signalsequenz des humanen Gewebe-Plasminogen-Aktivators (tPA).

7. Impfstoff gemäß einem der Ansprüche 1 bis 6, wobei das Plasmid eine Nucleinsäure enthält, die die ein Immunogen von BHV-1 codierende Nucleinsequenz sowie ein Stabilisationsintron umfasst.

8. Impfstoff gemäß Anspruch 7, in dem das Intron das Intron II des Beta-Globingens des Kaninchens ist.

9. Impfstoff, gemäß einem der Ansprüche 1 bis 8, der ein Plasmid umfasst, das eine das Glycoprotein gB von BHV-1 codierende Nucleinsäure enthält.

10. Impfstoff gemäß einem der Ansprüche 1 bis 8, der ein Plasmid umfasst, das eine das Glycoprotein gC von BHV-1 codierende Nucleinsäure enthält.

11. Impfstoff gemäß einem der Ansprüche 1 bis 8, der ein Plasmid umfasst, das eine das Glycoprotein gD von BHV-1 codierende Nucleinsäure enthält.

12. Impfstoff gemäß Anspruch 9, wobei die Sequenz des gB-Gens anstelle der Signalpeptidsequenz des Glycoproteins gB eine heterologe Signalsequenz umfasst, insbesondere die des tPA-Signals humanen Ursprungs, und/oder die Deletion des Teils des gB-Gens, der den transmembranen Bereich codiert.

13. Impfstoff gemäß Anspruch 10, wobei die Sequenz des gC-Gens anstelle der Signalsequenz des Glycoproteins gC eine heterologe Signalsequenz umfasst, insbesondere die tPA-Signalsequenz humanen Ursprungs, und/oder die Deletion des Teils des gC-Gens, der den transmembranen Bereich codiert.

14. Impfstoff gemäß Anspruch 11, wobei die Sequenz des gD-Gens anstelle der Signalpeptidsequenz des Glycoproteins gD eine heterologe Signalsequenz umfasst, insbesondere die tPA-Signalsequenz humanen Ursprungs, und/oder die Deletion des Teils des gD-Gens, der den transmembranen Bereich codiert.

15. Impfstoff gemäß Anspruch 1, umfassend ein Expressionsplasmid, das eine BHV-1-gB codierende Nucleinsäure umfasst, wobei das den transmembranen Bereich und den benachbarten C-terminalen Teil codierende Fragment deletiert wurde, sowie ein zweites Expressionsplasmid, das eine BHV-1-gD codierende Nucleinsäure umfasst, wobei das den transmembranen Bereich und den benachbarten C-terminalen Teil codierende Fragment deletiert wurde.


### LISTE DE SEQUENCES

<110> MERIAL
<120> Vaccin ADN pour animaux de rente
<130> ADNbpF
<140>
   <141>
<160> 106
<170> PatentIn Ver. 2.1
<210> 1
   <211> 40
   <212> ADN
   <213> Séquence artificielle
<220>
   <223> Description de la séquence artificielle: oligonucleotide
<400> 1
   gatctgcagc acgtgtctag aggatatcga attcgcggcc 40
<210> 2
   <211> 40
   <212> ADN
   <213> Séquence artificielle
<220>
   <223> Description de la séquence artificielle: oligonucleotide
<400> 2
   gatccgcggc cgcgaattcg atatcctcta gacacgtgct 40
<210> 3
   <211>20
   <212> ADN
   <213> Séquence artificielle
<220>
   <223> Description de la séquence artificielle: oligonucleotide
<400> 3
   ttggggaccc ttgattgttc 20
<210> 4
   <211> 21
   <212> ADN
   <213> Séquence artificielle
<220>
   <223> Description de la séquence artificielle: oligonucleotide
<400> 4
   ctgtaggaaa aagaagaagg c 21
<210> 5
   <211> 30
   <212> ADN
   <213> Séquence artificielle
<220>
   <223> Description de la séquence artificielle: oligonucleotide
<400> 5
   ctccatgtcg acttggggac ccttgattgt 30
<210> 6
   <211> 30
   <212> ADN
   <213> Séquence artificielle
<220>
   <223> Description de la séquence artificielle: oligonucleotide
<400> 6
   ctccatgtcg acctgtagga aaaagaagaa 30
<210> 7
   <211> 30
   <212> ADN
   <213> Séquence artificielle
<220>
   <223> Description de la séquence artificielle: oligonucleotide
<400> 7
   ttgtcgacat ggccgctcgc ggcggtgctc 30
<210> 8
   <211>21
   <212> ADN
   <213> Séquence artificielle
<220>
   <223> Description de la séquence artificielle: oligonucleotide
<400> 8
   gcagggcagc ggctagcgcg g 21
<210> 9
   <211> 52
   <212> ADN
   <213> Séquence artificielle
<220>
   <223> Description de la séquence artificielle: oligonucleotide
<400> 9
   ctgcacgagc tccggttcta cgacattgac cgcgtggtca agacggactg ag 52
<210> 10
   <211> 56
   <212> ADN
   <213> Séquence artificielle
<220>
   <223> Description de la séquence artificielle: oligonucleotide
<400> 10
   gatcctcagt ccgtcttgac cacgcggtca atgtcgtaga accggagctc gtgcag 56
<210> 11
   <211> 39
   <212> ADN
   <213> Séquence artificielle
<220>
   <223> Description de la séquence artificielle: oligonucleotide
<400> 11
   aaaatttcga tatccgccgc ggggcgaccg gcgacaacg 39
<210> 12
   <211> 33
   <212> ADN
   <213> Séquence artificielle
<220>
   <223> Description de la séquence artificielle: oligonucleotide
<400> 12
   ggaagatctt cagtccgtct tgaccacgcg gtc 33
<210> 13
   <211> 37
   <212> ADN
   <213> Séquence artificielle
<220>
   <223> Description de la séquence artificielle: oligonucleotide
<400> 13
   tcgtgcctgc ggcgcaaggc ccgggcgcgc ctgtagt 37
<210> 14
   <211> 37
   <212> ADN
   <213> Séquence artificielle
<220>
   <223> Description de la séquence artificielle: oligonucleotide
<400> 14
   ctagactaca ggcgcgcccg ggccttgcgc cgcaggc 37
<210> 15
   <211> 43
   <212> ADN
   <213> Séquence artificielle
<220>
   <223> Description de la séquence artificielle: oligonucleotide
<400> 15
   gcaccgctgc ccgagttctc cgcgaccgcc acgtacgact agt 43
<210> 16
   <211> 43
   <212> ADN
   <213> Séquence artificielle
<220>
   <223> Description de la séquence artificielle: oligonucleotide
<400> 16
   ctagactagt cgtacgtggc ggtcgcggag aactcgggca gcg 43
<210> 17
   <211> 39
   <212> ADN
   <213> Séquence artificielle
<220>
   <223> Description de la séquence artificielle: oligonucleotide
<400> 17
   aaaatttcga tatcccggcg ggggctcgcc gaggaggcg 39
<210> 18
   <211> 32
   <212> ADN
   <213> Séquence artificielle
<220>
   <223> Description de la séquence artificielle: oligonucleotide
<400> 18
   ggaagatctc tagtcgtacg tggcggtcgc gg 32
<210> 19
   <211> 33
   <212> ADN
   <213> Séquence artificielle
<220>
   <223> Description de la séquence artificielle: oligonucleotide
<400> 19
   tttctgcaga tgcaagggcc gacattggcc gtg 33
<210> 20
   <211> 31
   <212> ADN
   <213> Séquence artificielle
<220>
   <223> Description de la séquence artificielle: oligonucleotide
<400> 20
   tttctagatt agggcgtagc gggggcgggc g 31
<210> 21
   <211> 39
   <212> ADN
   <213> Séquence artificielle
<220>
   <223> Description de la séquence artificielle: oligonucleotide
<400> 21
   aaaatttcga tatcccccgc gccgcgggtg acggtatac 39
<210> 22
   <211> 33
   <212> ADN
   <213> Séquence artificielle
<220>
   <223> Description de la séquence artificielle: oligonucleotide
<400> 22
   ggaagatctt tagggcgtag cgggggcggg cgg 33
<210> 103
   <211> 36
   <212> ADN
   <213> Séquence artificielle
<220>
   <223> Description de la séquence artificielle: oligonucleotide
<400> 103
   catatcgtcg acatgtggct gcagaacctg cttctc 36
<210> 104
   <211> 34
   <212> ADN
   <213> Séquence artificielle
<220> <223> Description de la séquence artificielle: oligonucleotide
<400> 104
   catgaccaga tcttcacttc tgggctggtt ccca 34